# EUROPEAN PATENT SPECIFICATION

(11) **EP 1 578 494 B1**
(45) Date of publication and mention of the grant of the patent: **26.05.2010**
(21) Application number: 03787285.0
(22) Date of filing: 09.12.2003
(51) Int. Cl.: A61N 1/375, A61N 1/36

(54) **LEAD CONNECTION MODULE OF A MODULAR IMPLANTABLE MEDICAL DEVICE**
LEITUNGSVERBINDUNGSMODUL FÜR EINE MODULARE IMPLANTIERBARE MEDIZINISCHE VORRICHTUNG
MODULE DE CONNEXION DE FIL DE DISPOSITIF MEDICAL IMPLANTABLE MODULAIRE

(30) Priority: 09.12.2002 US 431854 P; 16.05.2003 US 471262 P; 20.09.2003 US 503945 P; 20.09.2003 US 503946 P; 01.10.2003 US 507857 P
(43) Date of publication of application: 28.09.2005
(73) Proprietor: Medtronic, Inc., Minneapolis, MN 55432-5604 (US)
(72) Inventor: SINGHAL, Ruchika, Minneapolis, MN 55401 (US); WAHLSTRAND, Carl, D., Lino Lakes, MN 55038 (US); JANZIG, Darren, A., Centerville, MN 55048 (US); SKIME, Robert, M., Coon Rapids, MN 55448 (US)
(74) Representative: von Kreisler Selting Werner
(86) International application number: PCT/US2003/038940
(87) International publication number: WO 2004/052457

(56) References cited:
- US-A- 5 645 586
- US-A- 6 016 449
- US-B1- 6 269 266
- US-B1- 6 308 101
- US-B1- 6 358 281
- US-B1- 6 480 743

## Description

The invention relates to medical devices, and more particularly, to implantable medical devices that deliver therapy to and/or monitor a patient.

Depending on the application for which they are implanted in a patient, implantable medical devices (IMDs) may include a variety of electrical and/or mechanical components. Typically, an IMD includes a rigid housing that houses all of its components, which are generally fragile, to protect the components from forces to which they would otherwise be exposed when implanted within the human body. In order to avoid potentially harmful interactions between the components and bodily fluids, e.g., corrosion, IMD housings are typically hermetically sealed. Many IMD housings are fabricated from Titanium because of its desirable rigidity and biocompatibility.
An example implantable medical device is described in US-B-6,358,281 which describes an implantable cochlear prosthesis that includes a housing defined by a gold coating, whereby the housing includes two sections. In this document, the housing is connected to cables that connect to a microphone casing and an electrode array. Another example implantable medical device is described in US-A-5,645,586 which describes an implantable device that includes a conforming housing. According to this document, the housing includes multiple segments that are interconnected at and pivot about hinge axes. The housing includes ports that allow connection of the device to sensing and defibrillation leads.
US-B-6,308,101 describes an implantable cochlear implant system that includes an implantable cochlear stimulator that is connected to an implantable speech processor via a cable. In addition, this document describes that the implantable cochlear stimulator can include two clamshell metal halves that are embedded in a silicone rubber encapsulant, as well as a metal header that includes electrical feedthroughs for connecting to an electrode array. US-B-6,269,266 describes an implantable device that includes a main module that includes electronics and a power supply module. The main module and the power supply module are connected via a coupling element.
The size and shape of an IMD housing is dependent on the sizes and shapes of the components of the IMD. Large components common to most IMDs include a battery, a telemetry coil, and a hybrid circuit that includes digital circuits, e.g., integrated circuit chips and/or a microprocessor, and analog circuit components. Attempts have been made to reduce the size of the IMD housing by reducing the size of these components, changing the shape of these components, and organizing these components within the IMD housing to avoid empty space within the housing. Despite these efforts to reduce the size of IMD housings, the size, shape and rigidity of IMD housings still greatly limits the locations within the human body where an IMD can be practically implanted.

Due to these limitations, an IMD is typically implanted within the abdomen, upper pectoral region, or interclavicular region of a patient. Leads or catheters must be used in order to deliver therapy or monitor a physiological parameter at a location of the body other than where the IMD is implanted. Implantation and positioning of leads and catheters can be difficult and time-consuming from the perspective of a surgeon, particularly where the IMD is located a significant distance from the treatment or monitoring site. Moreover, the increased surgical time, increased surgical trauma, and increased amount of implanted material associated with the use of leads and catheters can increase the risk to the patient of complications associated with the implantation of an IMD.

For example, IMDs that are used to treat or monitor the brain, e.g., to deliver deep brain stimulation (DBS) therapy, are implanted some distance away from the brain, e.g., within the interclavicular region of patients. The long leads that connect the implantable medical device to electrodes implanted within the brain require tunneling under the scalp and the skin of the neck, thereby requiring increased surgery and a prolonged amount of time under general anesthesia during the implant procedure. The lengthy tract along the leads is more susceptible to infection, and the leads can erode the overlying scalp, forcing removal so that the scalp can heal. Further, the long leads running under the scalp and through the neck are more susceptible to fracture due to torsional and other forces caused by normal head and neck movements.
The present invention provides an IMD according to claim 1. The dependent claims relate to individual embodiments of the invention.

In general, the invention relates to a lead connection module of a modular implantable medical device. In order to provide an implantable medical device with a smaller profile so that the IMD can better fit into available body locations, various functional components of the IMD are separated into individual interconnected modules. This modular architecture for the implantable medical device permits the device footprint to be distributed over a larger area while making the profile smaller. In addition, the multiple modules and their respective flexible interconnections may permit the overall shape of the implantable medical device to be formed to better match the body location into which it is to be implanted.

In a typical application, the component modules within the IMD are coupled to one or more leads that are deployed within a patient's body. External leads are used to electrically connect the external locations to a control module within the implantable medical device, and the control module can monitor the electrical activity, or regulate administration of therapy, or both. Accordingly, it is desirable for the external leads be both electronically and mechanically coupled to the implantable medical device. The present invention provides such a coupling of the external leads to the implantable medical device.

In one embodiment, the invention is directed to a an implantable medical device that includes at least two interconnected modules, each of the modules comprising a housing, and an overmold that at least partially encapsulates each of the housings. The overmold comprises a lead connection module that is configured to accept an external lead. The overmold is made of multiple materials, such as elastomeric and non-elastomeric materials.

In another embodiment, the invention is directed to an overmold for a modular implantable medical device. The overmold comprises a first material configured to hold at least part of a module, a second material coupled to the first material, and a lead connection module configured to accept an external lead, the lead connection module being deployed within the oversold.

The details of one or more embodiments of the invention are set forth in the accompanying drawings and the description below. Other features, objects, and advantages of the invention will be apparent from the description and drawings, and from the claims

FIGS. 1A and 1B are diagrams illustrating use of an implantable medical device in a patient according to an example embodiment of the present invention.

FIG. 2 is a schematic diagram illustrating an implantable medical device according to an embodiment of the present invention.

FIG. 3A and 3B are schematic diagrams illustrating an implantable medical device according to another embodiment of the present invention.

FIGS. 4A-4F are schematic diagrams illustrating various orientations of multiple modules within an implantable medical device according to various embodiments of the present invention.

FIG. 5A is a schematic diagram illustrating the construction of an overmold and modules used in construction of an implantable medical device according to the present invention.

FIG. 5B is an exploded view of an embodiment of the overmold and modules shown in FIG. 5A.

FIG. 5C is a side view of an embodiment of the overmold and modules shown in FIG. 5A.

FIGS. 6A-6B are schematic diagrams illustrating an example embodiment of a multi-module implantable medical device having multiple interconnect sites according to the present invention.

FIGS. 7A-7B are schematic diagrams illustrating an example embodiment of a multi-module implantable medical device having tethered interconnect sites according to the present invention.

FIG. 7C is a perspective view of a multi-module implantable medical device such as that depicted in FIGS. 7A and 7B.

FIGS. 8A-8B are schematic diagrams illustrating the interaction of components of an implantable medical device that are part of an overmold according to the present invention.

FIGS. 9A-9B are schematic diagrams illustrating an electronic module of a multi-module implantable medical device having a connection leads according to the present invention.

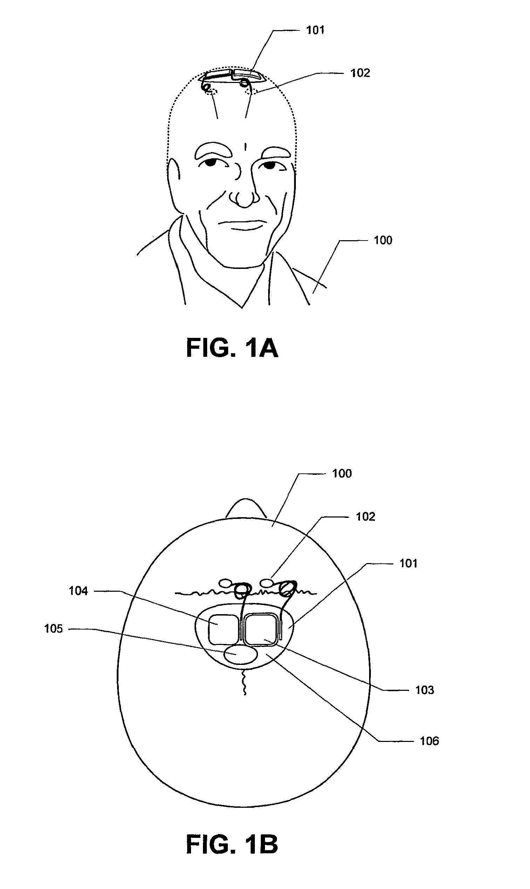

FIGS. 1A and 1B are diagrams illustrating use of an implantable medical device (IMD) in a patient according to an example embodiment of the present invention. An IMD101 is implanted within a patient 100 in order to permit IMD101 to provide therapies to the patient 100. In the example illustrated within FIGS. 1A-1B, IMD101 is implanted under the scalp of the patient 100 in order to locate the device 101 as close as possible to the location of leads 102 that provide the therapy.

FIG. 1A shows patient 100 with IMD 101 deployed beneath his scalp. In FIG. 1A, IMD 101 is a neurostimulator that provides deep brain stimulation via leads 102 deployed in the brain of patient 100. IMD 101 is deployed in proximity to site of stimulation therapy. IMD 101 may be used to treat any nervous system disorder including, but not limited to, epilepsy, pain, psychological disorders including mood and anxiety disorders, movement disorders (MVD) such as, but not limited to, essential tremor and Parkinson's disease and neurodegenerative disorders.

Although IMD 101 is depicted as a neurostimulator, the invention is not limited to applications in which the IMD is a neurostimulator. The invention may be employed with IMDs that perform any monitoring or therapeutic functions. The invention is not limited to IMDs that include leads deployed in the brain, but may also be employed with leads deployed anywhere in the head or neck including, for example, leads deployed on or near the surface of the skull, leads deployed beneath the skull such as near or on the dura mater, leads placed adjacent cranial or other nerves in the neck or head, or leads placed directly on the surface of the brain. Nor is the invention limited to IMDs that are coupled to electrodes. The invention may be employed with IMD coupled to any sensing or therapeutic elements, such as temperature sensors or motion sensors. The invention may also be employed with different types of IMDs including, but not limited to, IMDs operating in an open loop mode (also referred to as non-responsive operation), IMDs operating in a closed loop mode (also referred to as responsive), and IMDs for providing monitoring and/or warning.

In general, IMD 101 has a low profile, i.e., IMD 101 is thin to permit the IMD 101 to be deployed effectively, comfortably and cosmetically and under the scalp. In one embodiment of the invention, IMD 101 has a maximum thickness of between approximately 4 millimeters and approximately 8 millimeters. The use of a reduced profile may reduce the risk of infection, skin erosion and cosmetic issued related to the implantation of IMD 101.

Many locations within a patient do not present adequate profile for implantable medical devices. As such, many uses of such devices employ lengthy leads located remote from an implantation site of the IMD. The use of these lengthy leads requires complicated insertion procedures from the site of the IMD to the site of lead deployment that may cause medical complications to the patient as well as may lead to failures in connection leads. By constructing IMD 101 as a set of distributed modules connected together as described herein, IMD 101 may be deployed proximate to a treatment or monitoring site.

While the embodiment of IMD 101 shown in FIGS. 1A-1B is implanted under the scalp of patient 100 and may be used when the therapy provided to patient 100 includes neural stimulation of a brain, other embodiments of IMD 100 permit the device to be implanted at many other locations within the body. In addition, IMD 101 includes a plurality of interconnected modules. Each module generally perform assigned functions.

In the typical embodiment depicted in FIG. 1B, IMD 101 includes three modules, namely, a control module 103, a power supply module 104 and a recharge module 105. Control module 103 typically includes the electronic components associated with the functions of IMD 101. In a typical implementation, control module 103 may include a hybrid circuit that includes digital circuits such as integrated circuit chips and one or more microprocessors, and analog circuit components. Accordingly, control module 103 may also be referred to as an electronic module. Power supply module 104 typically comprises one or more energy storage devices, such as a rechargeable lithium ion battery. Recharge module 105 typically includes one or more coils for transmitting or receiving electromagnetic energy through the scalp. The transmitted energy may include energy to be stored in power supply module 104. In some embodiments, the transmitted energy may also include communication, such as information encoded in radio frequency transmissions.

Individual modules 103 and 104 may be encased in biocompatible metal shields such as titanium shield halves, and may be sealed against contamination. In addition, individual modules 103 and 104 may include insulation to electrically isolate the electrical components inside the modules from the metal shields. The modules are coupled to an overmold 106 which may be made of a biocompatible material. Use of the term "overmold" herein is not intend to limit the invention to embodiments in which the overmold is a molded structure. Overmold may be a molded structure, or may be a structure formed by any process.

In some embodiments of the invention, overmold 106 encases all modules 103, 104 and 105. In other embodiments, overmold 106 is disposed over or around the modules without encasing the modules. In further embodiments, overmold 106 acts as a "frame" to hold the modules in a fixed position relative to one another, but does not fully cover the modules. Some features of the overmold, and variations on the shape of the overmold, are presented below. In general, the shape of the overmold depends upon the arrangement of the modules. The overmold may be made of a variety of materials, such as flexible silicone. The overmold may also include a rigid polymer such as Ticothane surrounded by flexible silicone. The invention is not limited to these materials, however, and the overmold may comprise any combination of elastomeric and/or non-elastomeric materials.

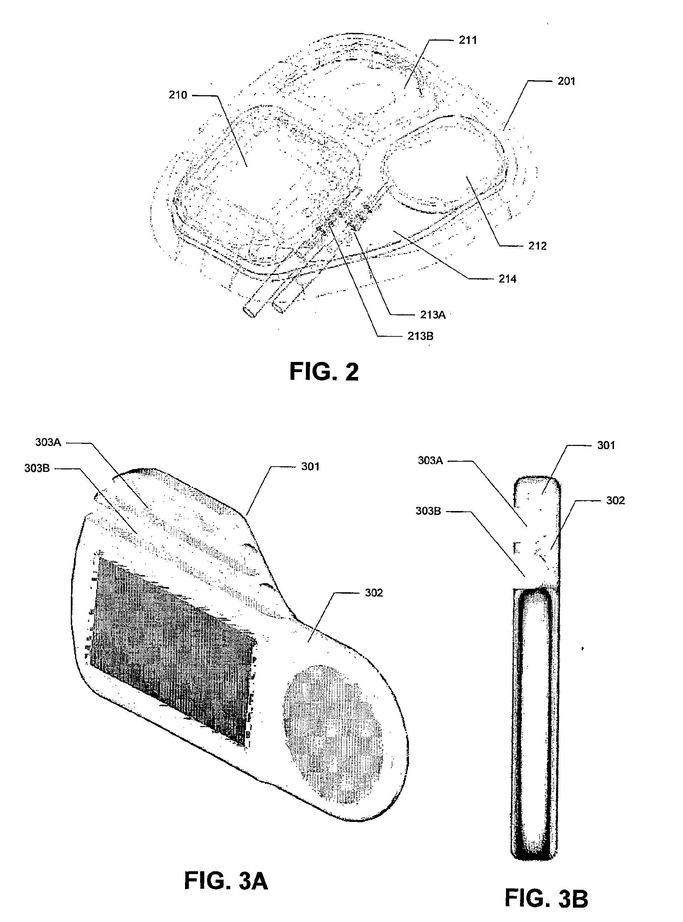

FIG. 2 is a schematic diagram illustrating an IMD according to another embodiment of the present invention. In this example embodiment, the IMD 201 is arranged in a triangular configuration, and includes three modules: a control module 210, a power supply module 211, and a recharge module 212. Overmold 214 at least partially covers the housings of control module 210 and power source supply module 211, and also at least partially covers recharge module 212. These three modules are physically connected together to construct IMD 201, and any modules may also be electrically coupled to one another.

IMD 201 also includes two lead connection modules 213A, 213B for accepting an external lead. In particular, overmold 214 includes lead connection modules 213A, 213B, and lead connection modules 213A, 213B need not be fixedly coupled to any module 210, 211 or 212. Lead connection modules 213A, 213B contain a set of lead connection modules that permits external leads to be connected to the IMD 210. In the case of electronic leads, for example, lead connection modules 213A, 213B include one or more conductors that electrically couple the external leads to control module 210. The triangular configuration of IMD 201 permits IMD 201 to possess a thin profile by spreading the modules over a larger surface area. The triangular shape also helps to keep the surface area compact. The structure of IMD 201 may also be curved to conform to the shape of the location within a patient in which the device is being implanted. For example, implantation of IMD 201 under the scalp of a patient may be accomplished if the overall shape of IMD 201 is curved to follow the shape of a patient's skull. Any number of shapes may be used to match a particular IMD 201 to an implantation location for a device.

FIG. 3A and 3B are schematic diagrams illustrating an IMD according to yet another embodiment of the present invention. In this embodiment of IMD301, a flat device is shown that consists of multiple modules. This embodiment of IMD 310 may be used in other locations within a patient in which the implantation location does not require such an exact match between the device and physical structures of the patient such as bone or muscle. IMD3 01 may provide a small profile when implanted as to not protrude excessively once implanted.

The flat embodiment shown in FIGS. 3A-3B may represent a device that may be a pectoral implant that may be used to treat angina, to provide vagal nerve stimulation, or to provide cardiac rhythm management. Similar devices may be implanted into an upper buttock implant location, into an abdomen location, and into periphery. A device implanted into an upper buttock location may be useful in urological and gastrological implantation therapies. A device implanted into an abdomen location may be useful in providing pain, spasticity, and chemotherapy treatment. A device implanted into a periphery location may be useful in providing muscle stimulation, on-site nerve stimulation, and diaphragm stimulation therapies.

IMD 301 comprises an overmold 302 that includes two lead connection modules 303A, 303B for accepting an external lead. In FIGS. 3A and 3B, lead connection modules 303A, 303B are depicted open to illustrate inclusion in overmold 302.

Additional alternate embodiments for implantable medical devices implemented according to principles of the present invention may also include non-electrical based therapies such as targeted introduction of fluids and similar therapeutic materials using pumps and reservoirs of material.

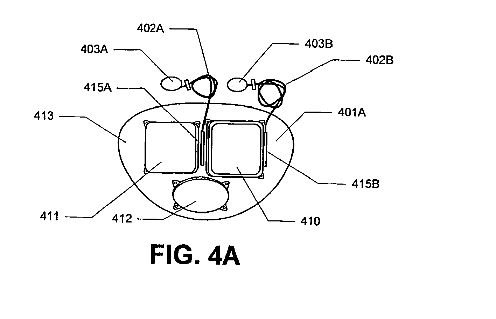

FIGS. 4A-4F are schematic diagrams illustrating exemplary configurations and orientations of modules within IMD 401A through 401 F (hereinafter 410), according to various embodiments of the present invention. IMD 401 consists of multiple modules that may be arranged into any number of orientations as shown in the various embodiments of FIGS. 4A-4F. For reference, each IMD 401 is depicted deployed proximate to the skull of a patient, with leads 402A and 402B deployed through burr holes 402A and 402B and coupled to IMD 401. The leads are coupled to the IMD via lead connection modules 415A and 415B. As shown in FIGS. 4A-4F, the lead connection modules may assume a variety of orientations relative to other components of IMD 401.

In each of these embodiments, IMD 401 has three modules as discussed above in reference to FIGS. 1B and 2: a control module 410, a power source module 411, and a recharge module 412. An overmold 413 at least partially covers the housings of control module 410 and power source module 411. The modules may be arranged into a number of orientations as long as any interconnections between the modules may be routed within the device. The various embodiments include triangular configurations, as is shown in FIGS. 4A-4C, or linear configurations as shown in FIGS. 4D-4F. In FIG. 4D, one of the three modules, such as the recharge module, is deployed as a tethered module 414 rather than being covered by overmold 413.

The invention is not limited to the deployments of the lead connection modules shown in FIGS. 4A-4F. The lead connection modules may be located on various positions within IMD 401. Lead connection modules may be oriented, for example, to permit the leads to be routed to lead locations in an efficient manner or to support management of excess lead length. Any number of other orientations and alternate embodiments may be constructed according to principles of the present invention and consistent with the claims recited herein.

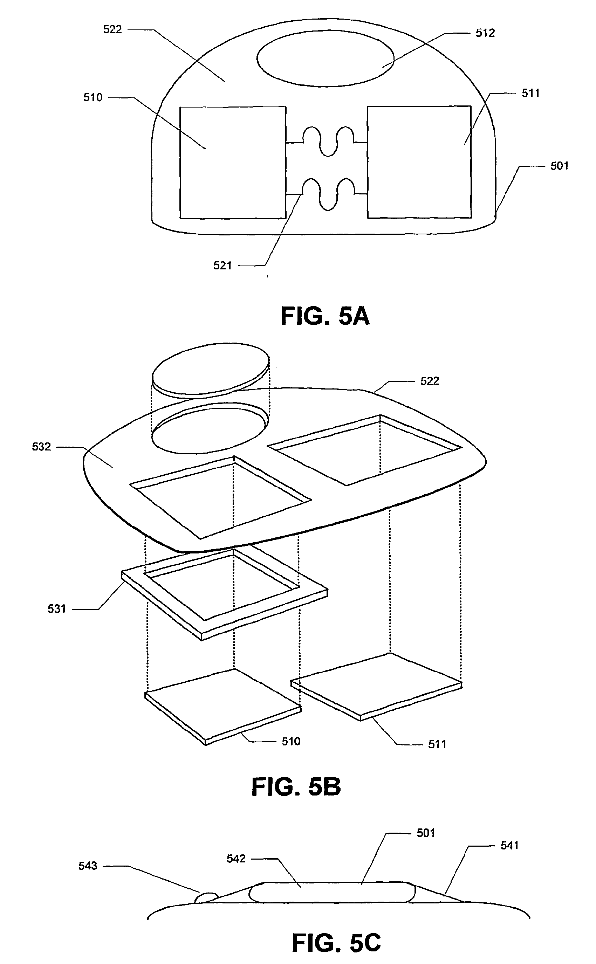

FIGS. 5A-5C are schematic diagrams illustrating an exemplary construction of an overmold used in construction of an IMD according to the present invention. FIG. 5A illustrates that IMD 501 comprises a set of modules 510-512, a set of motion restriction elements, such as motion restriction fibers 521. In FIG. 5A, motion restriction fibers 521 are coupled to modules 510 and 511, and are covered at least in part by overmold 522. Overmold 522 typically includes a solid biocompatible material. Overmold 522 may comprise an elastomeric material that is soft and flexible, such as silicone. In addition or in the alternative, overmold 522 may comprise a non-elastomeric material that imparts rigidity to IMD 501. In one embodiment, for example, a non-elastomeric material in overmold 522 acts as a "frame" to hold the modules in a fixed position relative to one another, and does not fully cover the modules. Overmold 522 covers, at least in part, the components and modules within IMD 501 while providing a flexible structure that permits the device 501 to conform to fit each individual patient. Because overmold 522 is typically flexible, IMD 501 may benefit from motion restriction devices such as motion restriction fibers 521, which provide structural integrity to device 501 once implanted into the patient.

FIG. 5B illustrates that the overmold 522 may include a non-elastomeric, or "hard" component 531 in addition to an elastomeric, or "soft" component 532. In FIG. 5B, the non-elastomeric component 531 is shaped to conform to the shape of at least one of modules 510-512 such that the modules may be restrained from motion by the non-elastomeric components. The non-elastomeric components 531 are typically made of a solid biocompatible material such as polysulfone, and may also be made of metal such as titanium.

The non-elastomeric components 531 are utilized in locations in which motion is to be restricted. Any or all modules may be constrained by one or more hard components 531. Overmold 522, including elastomeric and non-elastomeric components, can be fabricated into a single structure before the modules 510-512 are inserted into the device 501.

Generally, overmold 522 serves a number of functions. For example, overmold 522 incorporates motion restriction elements within the device 501, and attaches to modules and other elements to provide a unified device. In addition, overmold 522 provides a smooth interface surface for the device as it interacts with the patient, and protects electrical connections and feed through wires that connect modules to external leads.

Overmold 522 may also include a durometric specific material to provide desired device qualities such as flexibility and structural integrity. In addition, the material used to construct overmold 522 may possess a thermal conductivity characteristic to either act as a heat sink, or act as an insulator to shield the patient 100 from any excess heat from IMD 501. Because IMD 501 may be constructed from a large number of modules to perform a desired task, the materials selected for used in constructing the overmold 522 may vary as needed by each embodiment.

FIG. 5C illustrates that overmold 522 provides sloped interface 541 between an exemplary module 542 within IMD 501 and the patient's body. In embodiments in which IMD 501 is implanted within tight spaces, such as under the scalp of the patient, sloped interface 541 provides a smooth transition and eases sharp edges that are known to cause possible points of stress for tissue. An angle of interface from the patient's body and the sloped interface 541 can be approximately 135 degrees.

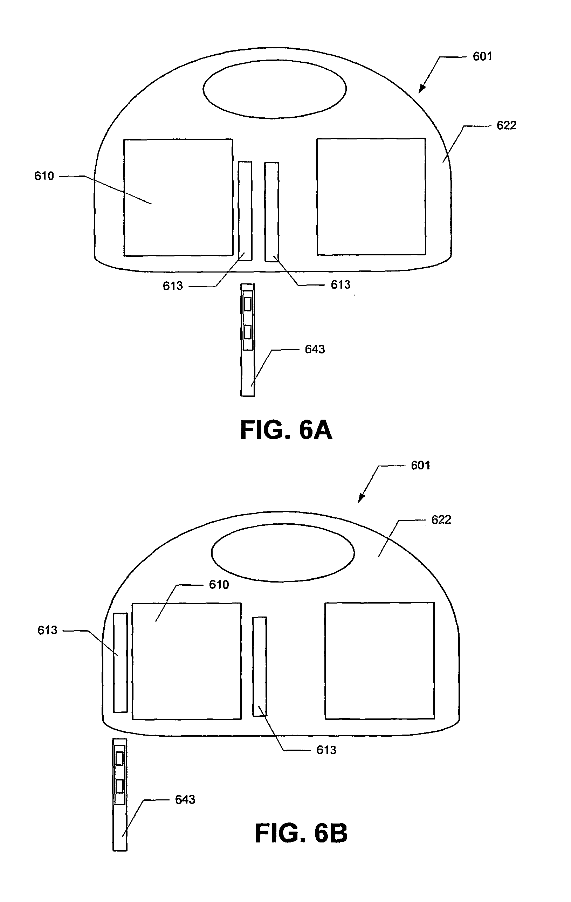

FIGS. 6A-6B are schematic diagrams illustrating an example embodiment of a multi-module IMD 601 having multiple interconnect sites according to the present invention. FIG. 6A shows a distributed IMD have multiple lead connection modules 613 that are located adjacent to each other while being near a control module 610. In contrast, FIG. 6B shows a distributed IMD have multiple lead connection modules 613 that are located on opposite sides of the control module 610. In FIGS. 6A and 6B, lead connection modules are included in overmold 622. The lead connection modules 613 provide a mechanism for electrically connecting electronics within control module 610 to one or more external leads 643. The external leads provide an electrical signal path from a desired part of the patient's body to IMD 601.

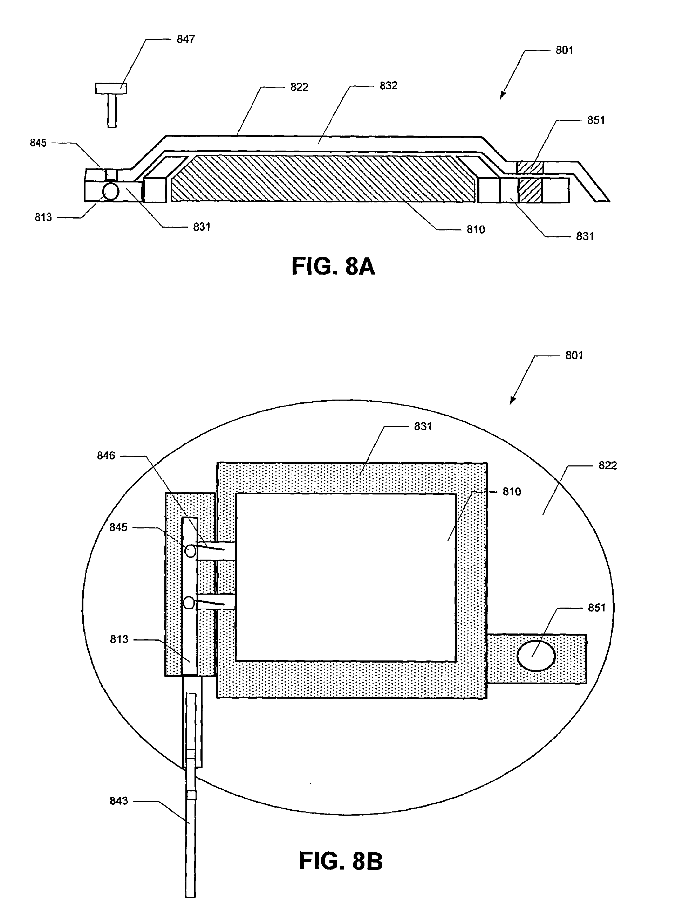

The embodiment shown is FIG. 6A is used when it is desirable for the external leads 643 to follow similar signal path lengths from the control module 610 to the treatment or monitoring site of the body. The embodiment of FIG. 6B may be used in cases in which the external leads 643 are farther apart. Because the lead connection modules 613 provide electrical connections between the control module 610 and the external leads 643, the lead connection modules 613 are typically located close to the control module 610 to reduce a length for electrical interconnections between them. Additional details regarding the interconnection of the control module 610, the lead connection modules 613, and the external leads 643 is discussed below in reference to FIGS. 8A-8B.

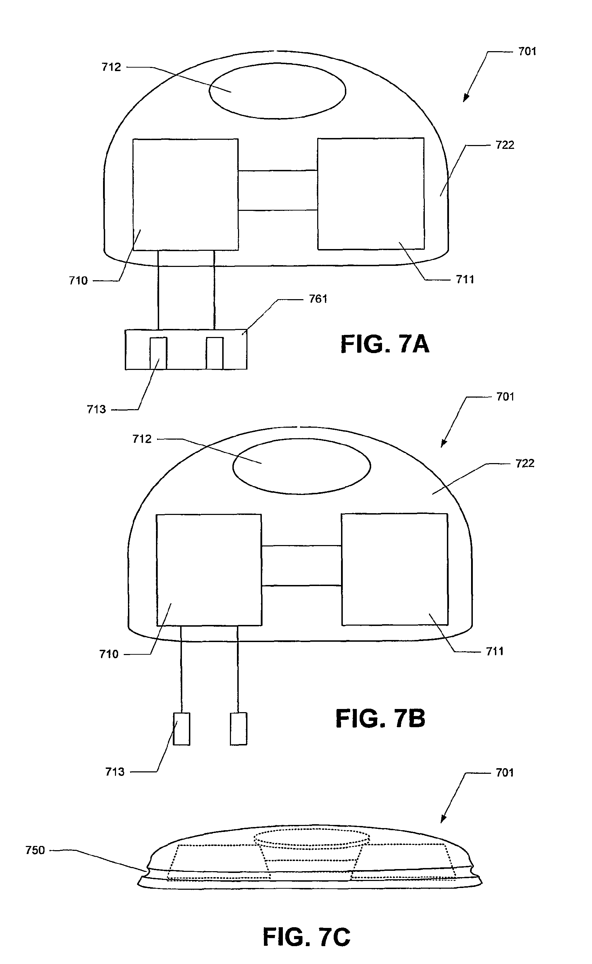

FIGS. 7A-7B are schematic diagrams illustrating an example embodiment of a multi-module IMD having tethered interconnect sites according to the present invention. In a first embodiment shown in FIG. 7A, an overmold 722 of an IMD 701 may cover and connect a plurality of modules 710-712 while not covering lead connection modules 713, which are part of a tethered interconnection housing 761. As shown in FIG. 7B, tethered interconnection housing 761 is optional.

In these embodiments, the lead connection modules 713 that are used to connect external leads (not shown) to the device 701 are not contained within the overmold 722. As such, the implantation of the device would not require the insertion of external leads into the 722. In addition, the external leads may be located a distance away from the overall device 701. Such an arrangement may assist in the management of the external leads as they are placed within the patient and routed to a lead location.

In an alternate embodiment shown in FIG. 7C, overmold 722 may include mechanical structures such one or more grooves or a pouch to contain and route the external leads and aid lead management. An exemplary structure, a groove 750, is depicted in a perspective view of IMD 701. In some implantations, external leads may possess an excess length that may be managed to reduce the risks of lead migration and lead damage.

FIGS. 8A-8B are schematic diagrams illustrating an exemplary interaction of components of an IMD 801. FIG. 8A provides a side view of an overmold 822, which includes one or more soft or elastomeric components 832 and one or more hard or non-elastomeric components 831, which interface with a control module 810. Non-elastomeric component 831 may be shaped to mate with the module 810 to provide motion restriction for the module. Non-elastomeric component 831 may be mechanically connected to other modules using a motion restriction device (not shown). The overmold 822 covers all of these components in this embodiment. A through hole 851 may be located through the non-elastomeric component 831 and elastomeric component 832 to provide an attachment point for IMD 801. In some embodiments, IMD 801 may be anchored in place using bone screws or other anchoring devices. Through holes 851 permit IMD 801 to be mechanically anchored to the patient once the device 801 is positioned at a desired location. In the embodiment shown in FIG. 8A, a bone screw inserted into through hole 851 would seat against non-elastomeric component 831, but the invention encompasses embodiments in which a bone screw would seat against another component, such as control module 810.

FIG. 8B illustrates a top view of the device 801 having elastomeric component 832 of overmold 822 covering the non-elastomeric components 831 that frame control module 810. The through hole 851 used as an attachment point is shown as part of non-elastomeric component 831 that is covered by elastomeric component 832. The shape of non-elastomeric component 831 and control module 810 are shown as being rectangular in this embodiment. However, one skilled in the art will recognize that any shape for the non-elastomeric component 831 and control module 810 may be used.

In both FIG. 8A and 8B, a lead interconnect device 813 is included within the non-elastomeric components 831 of overmold 822. In these examples, the non-elastomeric component 831 restrains control module 810 and external leads 843. Typically, the external leads have iso-diametric proximal ends for connection of the external leads 843 to IMD 801. An external lead 843 is inserted into the lead connection module in order to connect the external leads 843 to electronics within control module 810 of IMD 801. This electrical connection from the control module 810 to the external leads 843 is made using a module connection lead wire 846 that extends from control module 810 and physically connects with the external lead 843 within the lead connection module 813.

The lead connection module 813 may also include a mechanical lead securing mechanism 845 that engages the external lead 843 to restrain its motion and ensure electrical connection with feed-through wires 846. In the embodiment of FIG. 8A, a tool 847 is used to engage the mechanical lead securing mechanism 845 within the lead connection module 813. In this embodiment, the mechanical lead securing mechanism 845 comprises a mechanical set-screw that is tightened by a screwdriver. An example of such a mechanical lead securing mechanism 845 is a low-profile DBS lead extensions manufactured by Medtronic Inc. In alternate embodiments, the mechanical lead securing mechanism 845 may be tool-less using a variety of known securing technologies that ensures the external lead 843 does not separate from the lead connection module 813. Tool-assisted or tool-less coupling of leads to the IMD both allow medical personnel to couple leads to the IMD quickly and securely.

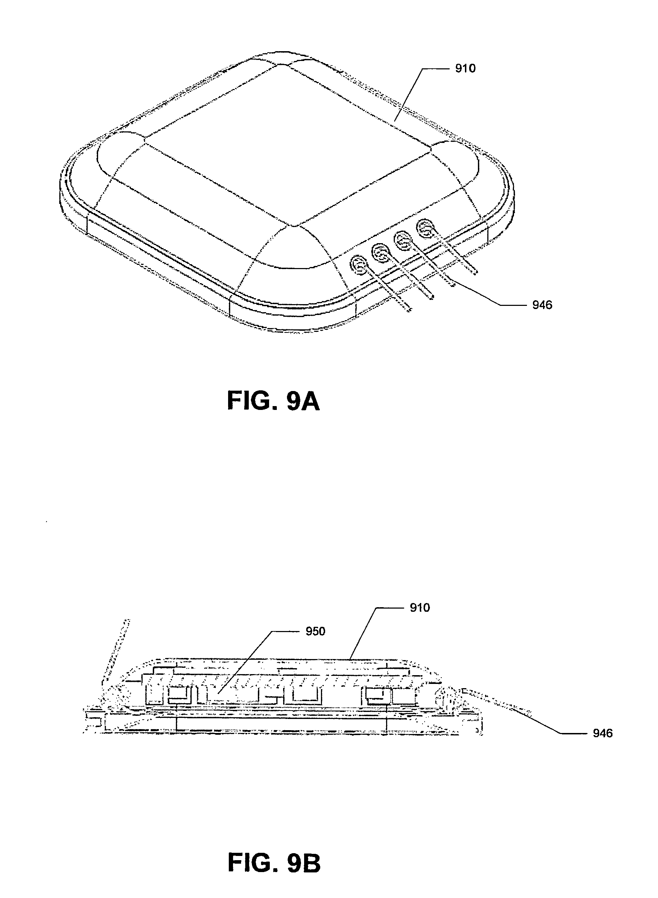

FIGS. 9A-9B are schematic diagrams illustrating a control module of a multi-module IMD having a connection leads according to the present invention. FIG. 9A provides a prospective view of a control module 910 to illustrate a set of feed-through wires 946 that electrically couple electronics 950 within the control module 810 to external leads as discussed above in reference to FIGS. 8A and 8B. Similarly, FIG. 9B provides a side view of the control module 910 of FIG. 9A to again illustrate the use of the feed-through wires 946 as discussed above.

## Claims

1. An implantable medical device (101, 201, 301, 401, 501, 601, 701, 801) comprising:
- at least two interconnected modules (103-105, 210-212, 410-412, 510-512, 610, 710-712, 810, 910), each of the modules comprising a respective one of at least two housings, and
- an overmold (106, 214, 302, 413, 522, 622, 722, 822) that at least partially encapsulates each of the housings, the overmold comprising a lead connection module (213A, 213B, 303A, 303B, 415A, 415B, 613, 813) configured to accept an external lead (102, 643, 843), wherein the overmold comprises a non-elastomeric first material (531, 831) and a second material (532, 832), and the lead connection module is deployed within the first material.

2. The implantable medical device of claim 1, wherein at least one module comprises a control module (103, 210, 410, 610, 810) containing electronic components.

3. The implantable medical device of claim 1, the lead connection module comprising at least one feed-through wire (846) to electrically couple an external lead (102, 643, 843) to an electronic component within the implantable medical device.

4. The implantable medical device of claim 1, wherein the lead connection module includes a mechanical lead securing mechanism (845).

5. The implantable medical device of claim 4, wherein the mechanical lead securing mechanism comprises a tool-less mechanical lead securing mechanism.

6. The implantable medical device of claim 1, wherein the implantable medical device has a maximum thickness of between approximately 4 millimeters and approximately 8 millimeters.

7. The implantable medical device of claim 1, wherein the lead connection module is configured to receive an iso-diametric external lead (843).

## Patentansprüche

1. Implantierbare medizinische Vorrichtung (101, 201, 301, 401, 501, 601, 701, 801) mit:
- mindestens zwei miteinander verbundene Module (103-105, 210-212, 410-412, 510-512, 610, 710-712, 810, 910) wobei jedes der Module jeweils eines von zwei Gehäuses aufweist; und
- einem Formteil (106, 214, 302, 413, 522, 622, 722, 822), das jedes der Gehäuse zumindest teilweise kapselt, wobei das Formteil ein Leitungsanschlussmodul (213A, 213B, 303A, 303B, 415A, 415B, 613, 813), das zum Aufnehmen einer externen Leitung (102, 643, 843) ausgebildet ist, wobei das Formteil ein nicht-elastomeres erstes Material (531, 831) und ein zweites Material (532, 832) aufweist und das Leitungsanschlussmodul in dem ersten Material angeordnet ist.

2. Implantierbare medizinische Vorrichtung nach Anspruch 1, bei welcher mindestens ein Modul ein Steuermodul (103, 210, 410, 610, 810) aufweist, das elektronische Bauteile aufweist.

3. Implantierbare medizinische Vorrichtung nach Anspruch 1, bei welcher das Leitungsanschlussmodul mindestens ein Verbindungskabel (846) zum elektrischen Verbinden einer externen Leitung (102, 643, 843) mit einem elektronischen Bauteil in der implantierbaren medizinischen Vorrichtung.

4. Implantierbare medizinische Vorrichtung nach Anspruch 1, bei welcher das Leitungsanschlussmodul einen mechanischen Leitungssicherungsmechanismus (845) aufweist.

5. Implantierbare medizinische Vorrichtung nach Anspruch 4, bei welcher der mechanische Leitungssicherungsmechanismus einen werkzeuglosen mechanischen Leitungssicherungsmechanismus aufweist.

6. Implantierbare medizinische Vorrichtung nach Anspruch 1, bei welcher die implantierbare medizinische Vorrichtung eine maximale Dicke zwischen ungefähr 4 Millimeter und ungefähr 8 Millimeter aufweist.

7. Implantierbare medizinische Vorrichtung nach Anspruch 1, bei welcher das Leitungsanschlussmodul zum Aufnehmen einer isodiametrischen externen Leitung (843) ausgebildet ist.

## Revendications

1. Dispositif médical implantable (101, 201, 301, 401, 501, 601, 701, 801) comprenant:
- au moins deux modules interconnectés (103-105, 210-212, 410-412, 510-512, 610, 710-712, 810, 910), chaque module respectivement comprenant un d'au moins deux boitiers,
- un élément surmoulé (106, 214, 302, 413, 522, 622, 722, 822) encapsulant au moins partiellement chaque boitier, ledit élément surmoulé comprenant un module de connexion de fil (213A, 213B, 303A, 303B, 415A, 415B, 613, 813) configuré pour recevoir an fil externe (102, 643, 843), dans lequel ledit élément surmoulé comprend un premier matériau non-élastomérique (531, 831) et un second matériau (532, 832), et ledit module de connexion de fil est arrangé dans ledit premier matériau.

2. Dispositif médical implantable selon la revendication 1, dans lequel au moins un module comprend un module de commande (103, 210, 410, 610, 810) comprenant des composants électroniques.

3. Dispositif médical implantable selon la revendication 1, dans lequel ledit module de connexion de fil comprend au moins un câble de connexion (846) pour coupler électriquement un fil externe (102, 643, 843) à un composant électronique dans ledit dispositif médical implantable.

4. Dispositif médical implantable selon la revendication 1, dans lequel ledit module de connexion de fil comprend mécanisme de fixation de fil mécanique (845).

5. Dispositif médical implantable selon la revendication 4, dans lequel ledit mécanisme de fixation de fil mécanique comprend un mécanisme de fixation de fil mécanique sans outillage.

6. Dispositif médical implantable selon la revendication 1, dans lequel ledit dispositif médical implantable a une épaisseur maximale d'environ 4 millimètres à environ 8 millimètres.

7. Dispositif médical implantable selon la revendication 1, dans lequel ledit module de connexion de fil est configuré pour recevoir un fil externe iso-diamétrique (843).
